(19) Europäisches Patentamt
European Patent Office
Office européen des brevets

(11) Veröffentlichungsnummer: **0 220 593**
**A1**

(12) # EUROPÄISCHE PATENTANMELDUNG

(21) Anmeldenummer: **86114183.6**

(22) Anmeldetag: **14.10.86**

(51) Int. Cl.⁴: **C 08 F 246/00**, C 08 F 226/04, C 12 N 11/08

(30) Priorität: **19.10.85 DE 3537259**

(43) Veröffentlichungstag der Anmeldung: **06.05.87 Patentblatt 87/19**

(84) Benannte Vertragsstaaten: **AT BE CH DE ES FR GB IT LI NL SE**

(71) Anmelder: **HOECHST AKTIENGESELLSCHAFT, Postfach 80 03 20, D-6230 Frankfurt am Main 80 (DE)**

(72) Erfinder: **Keil, Karl-Heinz, Dr., Lübecker Weg 3, D-6450 Hanau (DE)**
Erfinder: **Wullbrandt, Dieter, Dr., Bienerstrasse 29, D-6238 Hofheim am Taunus (DE)**
Erfinder: **Keller, Reinhold, Dr., Wiesenweg 5, D-6232 Bad Soden am Taunus (DE)**
Erfinder: **Engelhardt, Friedrich, Dr., Hünfelder Strasse 20, D-6000 Frankfurt am Main 61 (DE)**

(54) **Copolymerisat, Verfahren zu seiner Herstellung und seine Verwendung als Enzymträger.**

(57) Durch Suspensionspolymerisation von monomeren Oxiranylalkylverbindungen und/oder 2-Aziridinyl-alkylverbindungen, n-valenten Vernetzern, N-Vinylamiden, heterocyclischen 5-Ringverbindungen oder 6-Ringverbindungen, mit jeweils einer polymerisierbaren olefinischen Gruppe, und polymerisierbaren quartären Ammoniumsalzen enthält man ein poröses, perlförmiges Copolymerisat, das hervorragend zur Fixierung von Enzymen eingesetzt werden kann.

EP 0 220 593 A1

ACTORUM AG

HOECHST AKTIENGESELLSCHAFT Dr.Kh/gm HOE 85/F 235

# Copolymerisat, Verfahren zu seiner Herstellung und seine Verwendung als Enzymträger

Trägergebundene Enzyme haben eine Vielzahl von Anwendungen, z.B. in der medizinischen Analytik bei der Herstellung von Produkten aus Pharmazie und Pflanzenschutz sowie bei der Herstellung von Nahrungsmitteln. Die Vorteile der Verwendung von immobilisierten Enzymen bestehen in ihrer einfachen Abtrennung vom Substrat oder von Reaktionsprodukten ihrer, im Vergleich zur löslichen Form, oftmals erhöhten Stabilität, ihrer Wiederverwendbarkeit sowie der Möglichkeit, kontinuierliche Reaktionen in Säulen oder ähnlichen Reaktoren durchzuführen.

Es ist eine Vielzahl von Methoden zur Immobilisierung von Enzymen bekannt. Eine ausführliche Übersicht wird beispielsweise in Methods in Enzymology, Vol. XLIV, "Immobilized Enzymes", (Academic Press, 1976) und in J. Chibata: Immobilized Enzymes ( Kodansha Ltd., John Wiley & Sons, 1978) gegeben. Ein häufig beschriebener Weg ist die adsorptive, ionische oder kovalente Bindung von Enzymen an Träger.

In der DE-AS 2 237 316 werden als Trägersubstanzen quellbare, vernetzte Perlpolymerisate beschrieben, die durch Copolymerisation von reaktiven Gruppen enthaltenden Monomeren, vernetzenden Monomeren und hydrophilen Monomeren erhalten werden. Als reaktive Gruppe werden dabei die Halogenalkyl-, die Epoxid-, die Carbonsäurechlorid-, Carbonsäureanhydrid-, Carbonsäureazid-, Carbonsäurephenylester- und Hydroxamsäure-Gruppe offenbart. Diese Trägermaterialien haben jedoch eine Reihe von Nachteilen; so ist die Fixierung von Enzymen bei einigen von ihnen ziemlich langwierig; ihre Aktivität ist teilweise unbefriedigend und bei Verwendung der Anhydridvarianten kommt es außerdem zur Einführung von Ladungen.

In der deutschen Offenlegungsschrift 2 722 751 sowie die in der Europäischen Patentanmeldung 0 129 719 wird die Herstellung von Perlpolymerisaten auf Basis von (Meth)-Acrylamid-Derivaten zur Fixierung von Enzymen beschrieben. Diese Produkte zeigen jedoch eine zu große Hydrophobie gegenüber den hydrophilen Enzymsystemen, so daß die Fixierung nur unbefriedigend verläuft.

Es wurde nun gefunden, daß durch Einpolymerisation von quartären Stickstoffatomen das erfindungsgemäße Copolymerisat gegenüber Enzymsystemen eine hohe Selektivität besitzt.

Die Erfindung betrifft somit:

1) Ein vernetztes, poröses, perlförmiges Copolymerisat mit
   a) wiederkehrenden Einheiten der Verbindung der allgemeinen Formel I,

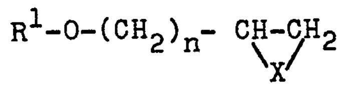

I

in der $R^1$ $(C_2-C_4)$ Alkenyl oder die korrespondierende Säure,
X Sauerstoff oder NH und
n 1-6 bedeuten

oder wiederkehrenden Einheiten der Verbindung der allgemeinen Formel II,

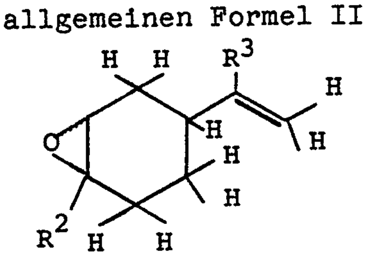

II

in der $R^2$ und $R^3$ unabhängig voneinander Wasserstoff oder Methyl bedeuten,

oder mehrer solcher Verbindungen,

b) wiederkehrenden Einheiten eines oder mehrerer n-valenter Vernetzer,

c) wiederkehrenden Einheiten der Verbindung der allgemeinen Formel III,

$$CH_2=CH-N(R^4)-C(R^5)=O \qquad III$$

in der $R^4$ und $R^5$ unabhängig voneinander Wasserstoff oder $(C_1-C_3)$-Alkyl bedeuten oder zusammen für $-(CH_2)_n-$ mit n = 3, 4 oder 5 stehen,
oder mehrerer derartiger Verbindungen,

d) wiederkehrenden Einheiten der Verbindungen der allgemeinen Formel IV,

$$R^6-X-(CH_2)_n-C(R^7)=C(R^8)-H \qquad IV$$

in der $R^6$ den Rest einer heterozyklischen 5-Ringverbindung, die im Ring mindestens ein protonierbares Stickstoffatom besitzt, oder Pyridyl, Chinolyl, Isochinolyl oder Pyrazinyl
$R^7$ und $R^8$ Wasserstoff oder $(C_1-C_4)$-Alkyl,
X Sauerstoff oder Schwefel oder eine direkte Bindung und n eine Zahl von 0 bis 4 bedeuten,
oder mehrerer derartiger Verbindungen,

e) wiederkehrenden Einheiten der Verbindung der allgemeinen Formel V,

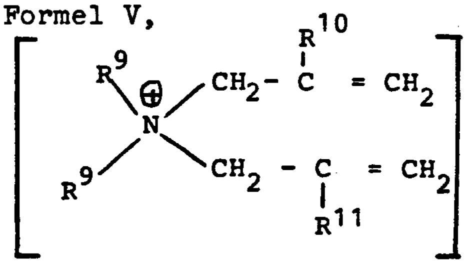 · $Y^{\ominus}$ V

in der $R^9$ eine $(C_1-C_{10})$-Alkylgruppe,
$R^{10}$ und $R^{11}$ unabhängig voneinander Wasserstoff oder Methyl und
Y Halogen bedeutet,
oder mehrere derartige Verbindungen.

2. Ein Verfahren zur Herstellung der erfindungsgemäßen, vernetzten, porösen, perlförmigen Copolymerisate, das dadurch gekennzeichnet ist, daß die unter 1 a-e genannten Verbindungen durch umgekehrte Suspensionspolymerisation polymerisiert werden.

3. Die Verwendung der erfindungsgemäßen vernetzten, porösen, perlförmigen Copolymerisate als Enzymträger.

Das erfindungsgemäße Copolymerisat ist aus 5 Monomerengruppen zusammengesetzt, die im folgenden als Gruppe A bis Gruppe E bezeichnet werden. Das Polymer setzt sich aus wiederkehrenden Einheiten dieser Monomeren zusammen. Unter Einheiten werden dabei im Copolymerisat statistisch verteilte Gruppen der Monomere verstanden.
Als Monomere aus der Gruppe A mit der allgemeinen Formel I können sowohl eine Oxiranylalkylverbindung wie auch eine 2-Aziridinylalkylverbindung mit einer Alkylgruppe bis zu 6 Kohlenstoffatomen eingesetzt werden, wobei eine Kettenlänge von 1 bis 4 C-Atomen bevorzugt ist. Der Oxiranylalkylrest bzw. der 2-Aziridinylalkylrest ist über ein Sauerstoffatom mit einer Alkenylgruppe von bis zu 4 Kohlenstoffatomen verbunden. Bevorzugt werden Glycidylacrylat, Glycidylmethacrylat, Allylglycidylether und Methallylglycidylether bzw. Dihydromyrcenoxid eingesetzt. Bevorzugte Monomere der allgemeinen Formel II, ebenfalls der Gruppe A zugeordnet, sind Vinylcyclohexenmonoxyd und Limonenoxid.

Die Monomere der Gruppe B haben die Funktion des Vernetzers bei der Herstellung der erfindungsgemäßen Mischpolymerisate. Sie verknüpfen n-Polymerenketten miteinander, wobei n eine Zahl gleich oder größer als 2 ist. Insbesondere ist n = 2, 3 oder 4, vorzugsweise 2.

Geeignete Vernetzer sind beispielsweise Verbindungen, die n-polymerisierbare Reste, insbesondere der Formeln $CH_2=CH-$, $CH_2=CH-CH_2-$ oder $CH_2=CH(R^{12})-CO-$ bzw. $CH_2=CH(R^{12})-CO-O$ im Molekül enthalten, wobei n die bereits genannte Bedeutung, normalerweise 2, 3 oder 4, vorzugsweise 2, besitzt und $R^{12}$ Wasserstoff oder $(C_1-C_4)$-Alkyl, insbesondere Wasserstoff oder Methyl, bedeuten. Beispiele für derartige Vernetzer sind: Triallyl-cyanurat, Triallylphosphat, N,N',N"-Trisacryloyl-perhydrotriazin, 1,2,3-Trivinyloxypropan, Tetraallyloxy-ethan, Pentaallylsaccharose, Triallyl-amin, N,N',N"-Tris-(2-acryloyloxy-ethyl- bzw. -methacryloyloxyethyl) -isocyanurat, Ethenphosphonsäure-di-allylester, Ethylenglykol-1,2-bis-(ethenphosphonsäureester) ferner allgemeine Verbindungen der Formeln VI, VII oder VIII

$$(CH_2=CH)_n-R^{13} \qquad VI$$
$$(CH_2=CH-CH_2)_n-R^{14} \qquad VII$$
$$(CH_2=CH-R^{12}-CO)_n-R^{15} \qquad VIII$$

worin $R^{12}$ die bereits genannte Bedeutung, n normalerweise 2, 3 oder 4, vorzugsweise 2, bedeutet und $R^{14}$ der durch Entfernung von n aciden H-Atomen entstandene Rest einer Di-, Tri-, Tetra- oder Polycarbonsäure und $R^{15}$ der durch Entfernung von n Hydroxyl-Wasserstoffatomen entstandene Rest eines Di-, Tri-, Tetra- oder Poly-ols und $R^{13}$ der durch Entfernung von n Wasserstoffatomen entstandene Rest eines aliphatischen, aromatischen oder heteroaromatischen Kohlenwasserstoffs darstellen.

Geeignete Vernetzer der Formel VI sind Divinyl-benzole, insbesondere 1,4-Divinylbenzol, Trivinylbenzole, Divinylpyridine, Divinylchinoline.

Beispiele für geeignete Vernetzer der Formel VII sind Triallyl-tricarballylat, Triallyl-aconitat, Triallyl-citrat, Tri-allyl-trimesinat, Triallyl-trimellitat, Diallyl-oxalat, Divinylphthalat, Diallylmaleat, -fumarat, -adipat, -phthalat.

Beispiele für geeignete Vernetzer der Formel VIII sind Trimethylolpropan-tri-acrylat und -methacrylat, Pentaerythrittetra-acrylat und -methacrylat, Pentaerythrit-triacrylat und -methacrylat, Dimethylenglykoldiacrylat- oder -dimethacrylat, Butylenglykoldiacrylat, oder -dimethacrylat.

Von den Verbindungen der allgemeinen Formel VIII sind besonders geeignet die Diacrylate und insbesondere die Dimethylacrylate des Ethylenglykols, des Diethylenglykols und der Polyethylenglykole 200 bis 600, insbesondere die Dimethacrylate der Polyethylenglykole 200 bis 600.

Geeignete Vernetzer sind ferner z.B. Diallylamin, Divinylketon, Divinylsulfon, Diallylmelamin.

2-valente Vernetzer sowie wasserlösliche Vernetzer sind bevorzugt. Geeignete wasserlösliche Vernetzer sind insbesondere Derivate der Acryl- bzw. Methacryl-säure, wie z.B. N,N'-Methylen-bis-acrylamid, N,N'-Methylen-bis-methacrylamid, N,N'-Methylen-bis-(N-hydroxymethyl-methacryl-amid), N,N'-Bis-(methacryloyl)-aminoessigsäure, N,N'-Bis-(acryloyl)-aminoessigsäure, 1,2-Bis-(acrylamido)-1,2-dihydroxy-ethan, 1,2-Bis-(methacrylamido)-1,2-dihydroxy-ethan, 1,2-Bis-(N-hydroxymethyl-methacrylamido)-1,2-dihydroxy-ethan, 1,2-Bis-(N-methoxymethyl-methacrylamido)-1,2-dimethyloxy-ethan, 1,6-Bis-(acrylamido)-hexan, 1,6-Bis-(methacrylamido)-hexan, 2-Methyl-1,4-bis-(acrylamido)-butan ("Isovalerilyden-bis-acrylamid"), 2-Methyl-1,4-bis-(methacrylamido)-butan ("Isovalerilyden-bis-methacrylamid"), die Diacrylate und Dimethacrylate des Ethylenglykols, Diethylenglykols und der Polyglykole 200 bis 600.

Günstig sind auch Kombinationen von zwei oder mehreren Vernetzern, z.B. von N,N'-Methylen-bis-acrylamid/N,N'-Bis-(acryloyl)-aminoessigsäure; N,N'-Methylen-bis-acrylamid/ Isovalerilyden-bis-acrylamid; N,N'-Methylen-bis-acrylamid/ Ethylenglykol-1,2-bis-(ethenphosphonsäureester). Bevorzugte Vernetzerkombinationen können als eine Vernetzerkomponente nicht nur N,N'-Methylen-bis-acrylamid und/oder N,N'-Methylenbis-methacrylamid, sondern auch die Diacrylate und/ oder Dimethacrylate des Ethylenglykols, Diethylenglykols oder der Polyethylenglykole 200 bis 600 oder N,N',N"-Tris(2-acryloyloxyethyl)-isocyanurat oder N,N',N"-Tris-(2-methacryloyloxyethyl)-isocyanurat enthalten. Besonders bevorzugte Vernetzerkombinationen enthalten oder bestehen aus N, N'-Methylen-bis-acrylamid/Dimethacrylat des Polyethylenglykols 200 bis 600 und/oder des entsprechenden Methacrylamids und/oder des entsprechenden Diacrylats.

Geeignete n-valente Vernetzer sind jedoch auch polymerisierbare silizium- oder bororganische Verbindungen, insbesondere solche Verbindungen, die nur eine oder zwei polymerisierbare olefinische Doppelbindungen im Molekül enthalten, die aber aufgrund von Sekundärreaktionen zusätzlich (n-1) bzw. (n-2) Polymerenketten miteinander verknüpfen können. Derartige Vernetzer sind z.B. Alkoxy-Gruppen enthaltende Silane, bei denen die Alkoxy-Gruppen in wäßrigem Medium intermediär zu Si-OH-Gruppen hydrolysiert werden. Zwei derartige Silanolgruppen bewirken dann unter Kondensation und Ausbildung einer Siloxanbindung Si-O-Si die Verknüpfung zweier Ketten. Geeignete derartige Vernetzer sind siliziumorganische Verbindungen, soweit sie 1 oder 2 olefinische Doppelbindungen und (n-1) bzw. (n-2) Alkoxygruppen enthalten. Von den polymerisierbaren silizium- oder bor-organischen Verbindungen sind die polymerisierbaren siliziumorganischen Verbindungen bevorzugt. Geeignete polymerisierbare silizium-organische Verbindungen sind z.B. polymerisierbare olefinische Gruppen enthaltende Silane, Siloxane und Silazane. Bevorzugt sind Acryl- bzw. Methacrylsäure-3-(trimethoxy-silyl)-propyl-ester, Acryl- bzw. Methacrylsäure-3-(triethoxy-silyl)-propyl-ester, Acryl- bzw. Methacrylsäure-3-(tri(methoxyethoxy)-silyl)-propylester, Acryl- bzw. Methacrylsäure-3-(tri(methoxy-ethoxy-ethoxy)-silyl)-propylester, Acryl- bzw. Methacrylsäure-3-(di-(methoxy)-methyl-silyl)-propyl-ester, Acryl- bzw. Methacrylsäure-3-(di-(ethoxy)-ethyl-silyl)-propylester, Vinyl-tri-ethoxy-silan, Vinyl-tri-methoxy-silan, Vinyl-tri-methoxy-silan, Vinyl-tri-allyloxy-silan, Allyltri-allyloxy-silan, Vinylmethyl-diethoxy-silan, Vinylmethyl-di-methoxy-silan, Vinyl-tri-acetoxy-silan, Vinyl-tri-(methoxyethoxy)-silan, 1,3-Di-vinyl-1,1,3,3-tetra-methyl-disiloxan, 1,3-Di-vinyl-1,1,3,3-tetra-methyl-disilazan, $CH_2=CH-COO-(CH_2)_3-[Si-(CH_3)_2-O]_p-Si(CH_3)_2-(CH_2)_3-O-CO-CH=CH_2$, $CH_2=C(CH_3)-COO-(CH_2)_3-[Si(CH_3)_2-O]_p-Si(CH_3)_2-(CH_2)_3-O-CO-C(CH_3)=CH_2$, wobei p eine Zahl von 1 bis 20, insbesondere eine Zahl von 1 bis 10, bedeutet, $CH_2=CH-CH_2-NH-SiH(CH_3)-N(CH_2CH=CH_2)-SiH(CH_3)NH-CH_2-CH=CH_2$.

Geeignete bororganische Verbindungen sind beispielsweise Triallyl-borsäureester und Trimethallyl-borsäureester.

Die Monomere der Gruppe C sind Vinylamide der allgemeinen Formel III.
Geeignete N-Vinyl-amide sind z.B. N-Vinyl-formamid, N-Vinylacetamid, N-Vinyl-N-methyl-acetamid, N-Vinyl-N-methylformamid, N-Vinyl-N-ethyl-acetamid, N-Vinyl-propionamid, N-Vinyl-N-methyl-propionamid, N-Vinylethyl-propionamid, N-Vinyl-butyramid, N-Vinyl-N-methyl-butyramid, N-Vinyl-N-ethylbutyramid, N-Vinyl-pyrrolidon und N-Vinyl-caprolactam. Besonders geeignet sind N-Vinyl-formamid und insbesondere N-Vinyl-N-methyl-acetamid.

Die Monomere der Gruppe D sind heterocyclische 5-Ringverbindungen oder 6-Ringverbindungen, wie Pyridyl-, Chinolyl-, Isochinolyl- oder Pyrazinyl-Verbindungen, mit jeweils einer

polymerisierbaren olefinischen Gruppe, wie sie in der allgemeinen Formel IV dargestellt sind.
Die polymerisierbare olefinische Gruppe ist in der Regel direkt, gegebenenfalls aber auch indirekt, z.B. über ein Sauerstoff- oder Schwefelatom, an den Kern gebunden. Für die Reste $R^7$, $R^8$ ist die Kombination H/$CH_3$ bzw. $CH_3$/H oder H/H bevorzugt. Für n ist 1, insbesondere aber O bevorzugt. Polymerisierbare olefinische Gruppen sind insbesondere die Vinyl-, Allyl-, Methallyl- oder die Isopropenylgruppe. Stellt der $R^6$ den Rest einer heterocyclischen 5-Ringverbindung dar, so kann diese z.B. ein, zwei, drei oder vier Stickstoffatome besitzten, von denen mindestens eines protonierbar sein muß, wobei der heterocyclische Ring gegebenenfalls auch noch ein anderes Heteroatom oder mehrere andere Heteroatome, insbesondere Sauerstoff und/oder Schwefel enthalten kann. Protonierbare Stickstoffatome sind basische Stickstoffatome mit einem freien Elektronenpaar, die z.B. in dem 5-Ring als -N=, -NH- oder -N- eingebaut sind. Stickstoffatome, die z.B. einer Ketogruppe benachbart sind, besitzen keine basischen Eigenschaften mehr und können daher nicht mehr protoniert werden. Der heterocyclische 5-Ring kann gesättigt, teilweise gesättigt oder ungesättigt sein und beispielsweise ein Imidazol, Imidazolin, Oxazol, Oxazolin, Oxazolidin, Thiazol, Oxadiazol, Pyrrol, Triazol oder Tetrazol darstellen und kann gegebenenfalls noch eine oder mehrere ($C_1$-$C_4$)Alkyl- und/oder ($C_2$-$C_4$) Hydroxyalkylgruppen besitzen.

Als eine polymerisierbare olefinische Gruppe enthaltende heterocyclische 5-Ringverbindung, die im Ring mindestens ein protonierbares Stickstoffatom besitzt, sind z.B. zu nennen: 1-Vinyl-1,2,3-triazol, 1-Vinyl-1,2,4-triazol, 4-Vinyl-1,2,3-triazol, 5-Vinyl-1,2,3-triazol, 1-Vinyl-1,2,3,4-tetrazol, 2-Vinyl-1,2,3,4-tetrazol, 2-Allyl-1,2,3,4-tetrazol, 1-Allyl-1,2,3,4-tetrazol, 1-Methyl-5-vinyl-1,2,3,4-tetrazol, 2-Methyl-5-vinyl-1,2,3,4-tetrazol, 3-Vinyl-1,2,4-oxadiazol, 3-Vinyl-5-methyl-1,2,4-oxadiazol, 3-Isopropenyl-1,2,4-oxadiazol, 2-Isopropenyl-1,3,4-oxadiazolin-5-on, 3-Isopropenyl-1,2,4-oxadiazol, 3-Isopropenyl-5-methyl-1,2,4-oxadiazol, 2-Vinyl-oxazolin, 2-Isopropenyl-oxazolin, 2-Vinyl-3-methyl-oxazolidin, 2-Vinyl-thiazol, 4-Vinyl-thiazol, 1-Vinyl-2-imidazolin, 2-Vinyl-4-(oder 5-)-methyl-2-imidazolin, 1-Vinyl-2-methyl-2-imidazolin, 1-Vinyl-imidazol, 1-Methyl-2-vinyl-imidazol, 1-Vinyl-2-methyl-imidazol, 1-Vinyl- 4-(2-hydroxyethyl)-imidazol, N-Vinyl-pyrrol, 2-Isopropenyl-2-imidazolin, 2-Vinyl-3-methyl-2-imidazolin, 1-Vinyl-2,4-dimethyl-imidazol. Die Vinyl-imidazole, insbesondere das 1-N-Vinyl-imidazol sind bevorzugt.

Stellt $R^6$ den Rest einer Pyridyl-, Chinolyl-, Isochinolyl- oder Pyrazinylverbindung dar, so besitzt sie, falls sie weitere Substituenten enthält, insbesondere einen, zwei oder drei $(C_1-C_4)$Alkyl- und/oder OH-Substituenten. Besonders bevorzugt sind ein oder zwei Alkylreste oder eine OH-Gruppe oder eine OH-Gruppe und ein Alkylrest als Substituenten.

Geeignete Verbindungen einer, eine polymerisierbare olefinische Gruppe enthaltende Pyridyl-, Chinolyl-, Isochinolyl- oder Pyrazinyl-Verbindung sind beispielsweise: 2-Vinyl-pyridin, 3-Vinyl-pyridin, 4-Vinyl-pyridin, 3-Isopropenylpyridin, 2-Vinyl-5-methyl-pyridin, 2-Methyl-5-vinyl-pyridin, 3-Methyl-5-vinyl-pyridin, 2,4-Dimethyl-6-vinyl-pyridin, 3-Methyl-4-vinyl-pyridin, 3-Ethyl-4-vinyl-pyridin, 3-Methyl-2-vinyl-pyridin, 3-Ethyl-2-vinyl-pyridin, 4-Methyl-4-vinyl-chinolin, 2-Methyl-5-vinyl-chinolin, 1-Methyl-5-vinyl-isochinolin, 2-Isopropenyl-chinolin, 2-Vinylpyrazin, 2-Vinyl- 5-ethyl-pyridin, 2-Vinyl-4,6-dimethyl-pyridin, 2-Vinyl-chinolin, 2-Methyl-3-vinyl-8-hydroxy-chinolin. Von den Pyridyl-, Chinolyl-, Isochinolyl- oder Pyrazinyl-Verbindungen sind die Pyridyl-Verbindungen, insbesondere Verbindungen der Vinylpyridine, vor allem das 4-Vinyl-pyridin, bevorzugt.

Die Monomere der Gruppe E sind polymerisierbare quartäre Ammoniumsalze der allgemeinen Formel V. Bevorzugt sind Verbindungen, in denen $R^9$ eine $(C_1-C_5)$-Alkylgruppe darstellt. Besonders bevorzugt sind die Verbindungen: Diallyl-dimethyl- ammonium-chlorid, Diallyl-diethyl-ammoniumchlorid, Diallyl-di-n-propylammoniumchlorid, Diallyl-di-tertiärbutyl-ammoniumchlorid, Diallyl-di-n-butyl-ammonium-chlorid, Diallyl-di-n-pentyl-ammoniumchlorid, Diallyl-diisopentylammoniumchlorid.

Die erfindungsgemäßen vernetzten, porösen, perlförmigen Copolymerisate werden durch Perlpolymerisation von 10-50 Gew.-%, bevorzugt 25-75 Gew.-%, des Monomeranteils A (Verbindung der allgemeinen Formel I oder II), 0,05-50 Gew.-%, bevorzugt 0,1-35 Gew.-% des Monomeranteils B (n-valente Vernetzer), 0-50 Gew.-%, bevorzugt 5-25 Gew.-%, des Monomeranteils C (Verbindung der allgemeinen Formel III), 0-90 Gew.-%, bevorzugt 10-80 Gew.-%, des Monomeranteils D (Verbindung der allgemeinen Formel IV) und 95-80 Gew.-%, bevorzugt 10-50 Gew.-%, des Monomeranteils E (Verbindung der allgemeinen Formel V) hergestellt. Bei der Copolymerisation können bis zu 50 Gew.-% der eingesetzten Pyridyl-, Chinolyl-, Isochinolyl- oder Pyrazinyl-Verbindung durch eine oder mehrere der bereits genannten heterocyclischen 5-Ringverbindungen mit einer polymerisierbaren olefinischen Gruppe und mindestens einem protonierbaren Stickstoffatom ersetzt werden. Die polymerisierbaren Monomeren können in der Regel in handelsüblicher Form, also ohne vorherige Reinigung, eingesetzt werden.

Die Copolymerisation erfolgt nach dem Verfahren der Perlpolymerisation (vgl. z.B. Houben-Weyl: Methoden der organischen Chemie, 4. Auflage, Band 14,1 (1961), Makromolekulare Stoffe, Teil 1, Seite 406 ff). Bei der Perlpolymerisation wird das zu polymerisierende Monomerengemisch in der Regel in Wasser oder einer anderen Flüssigkeit, in der die Monomeren und das entstehende Copolymerisat unlöslich sind,

durch mechanisches Rühren oder Schütteln, vorzugsweise unter Mitwirkung einer oder mehrerer geeigneter Dispergatoren zu der gewünschten Perlgröße zerteilt und anschließend copolymerisiert. Die Copolymerisation wird in an sich bekannter Weise, z.B. durch UV-Licht, energiereiche Strahlung, in der Regel aber durch einen im Monomerengemisch löslichen, Radikale liefernden Initiator ausgelöst. Geeignete Initiatoren sind z.B. Benzoylperoxid, tert-Butylhydro-peroxid, Cumol-peroxid, Methylethylketon-peroxid, Lauroyl-peroxid, tert-Butylperbenzoat, tert-Butyldiperphthalat, Azodiisobutyronitril, 2,2'-Azobis-(2,4-dimethylvaleronitril), 2-Phenyl-azo-2,4-dimethyl-4-methoxy-valeronitril, 2-Cyano-2-propyl-azoformamid, Azodiisobutyramid, Dimethyl-, Diethyl- oder Dibutyl-azobis-methylvalerat. Bezogen auf die Monomerenmenge (einschließlich Vernetzer) werden etwa 0,01 bis 2 Gew.-%, vorzugsweise 0,1 bis 1 Gew.-% Initiator angewandt.

Als Dispergatoren werden wasserlösliche oder wasserlöslich gemachte Naturstoffe, insbesondere Kohlenhydrate und Eiweißstoffe, z.B. löslich gemachte Stärke, Methylstärke, Methylcellulose oder andere Celluloseether, Methylhydroxypropylcellulose, Celluloseglykolat, ferner Cholesterin, Saponin, Leim, Tragant etc. oder wasserlösliche hochmolekulare synthetische Emulgatoren, wie z.B. Polyvinylalkohol, Polyacrylate oder Polymethacrylate in Mengen von 0,05 bis 3 Gew.%, vorzugsweise 0,1 bis 1 Gew.-%, bezogen auf die vorhandene Wassermenge, verwendet.

Es ist zweckmäßig, die Perlpolymerisation in Gegenwart von Salzen, wie z.B. Natriumchlorid oder Natriumnitrit, insbesondere von Natriumformiat, durchzuführen, die in der wässrigen Phase aufgelöst werden. Ferner ist es zweckmäßig, die Polymerisation unter Ausschluß von Sauerstoff durchzuführen. Dies kann z.B. in bekannter Weise durch Spülen bzw. Durchleiten eines Inertgases, wie z.B. Stickstoff, erfolgen. Die Perlpolymerisation wird normalerweise bei Temperaturen

von 45 bis 95°C, vorzugsweise 55 bis 85°C, durchgeführt und ist in der Regel nach 0,3 bis 3 h beendet. Nach beendeter Polymerisation wird das erhaltene perlförmige Copolymerisat abgetrennt, mit Wasser oder einem organischen Lösungsmittel gewaschen und getrocknet.

Das erfindungsgemäße vernetzte Copolymerisat liegt in Form poröser Perlen vor, deren Durchmesser durch die Wahl der Herstellungsbedingungen im Bereich von ca. 2 bis 0,04 mm wählbar ist. Bei gegebenen Herstellungsbedingungen werden Perlen mit einem engen Durchmesserspektrum erhalten. Das erfindungsgemäße Copolymerisat liegt insbesondere in Form von Perlen mit einem Durchmesser von 0,02 bis 0,2 mm, vorzugsweise 0,02 bis 0,1 mm, vor und ist bevorzugt zur dauerhaften Immobilisierung von Enzymen geeignet, wobei deren Aktivität erhalten bleibt. Der Träger ist problemlos zu handhaben, d.h. er kann ohne Vorsichtsmaßnahmen gelagert werden. Außerdem besitzt er gute mechanische und hydrodynamische Eigenschaften.

Zur Immobilisierung von Enzymen wird das Copolymerisat vorteilhaft mit einem Diamin und einem Dialdehyd, bevorzugt Glutardialdehyd, aktiviert. Es werden insbesondere Diamine eingesetzt, deren entständige Aminogruppen über Alkylgruppen bzw. Alkyl-Benzylgruppen miteinander verbunden sind. Besonders bevorzugt sind Alkylgruppen mit einer Kettenlänge von 2, 4 oder 6 Kohlenstoffatomen. Das Beladen der Träger geschieht mit einer wäßrigen Enzymlösung, die je nach Art des Enzyms zweckmäßig einen Puffer enthält. Lipasen lassen sich besonders gut immobilisieren. Der Puffer wird vorzugsweise in einer Molarität von 0,05-0,5, insbesondere von 0,1-0,2 ein gesetzt. Der Träger wird in der Enzymlösung bei Raumtemperatur unter Rühren 10-20 Stunden, vorzugsweise 14-18 Stunden, inkubiert. Nach Abtrennen von der wäßrigen Lösung kann das Copolymerisat dann als Biokatalysator eingesetzt werden.

In den folgenden Beispielen wird die Erfindung detailliert beschrieben. Die Prozentangaben beziehen sich auf das Gewicht.

Beispiel 1:

In einem 2 Liter 4-Halskolben, der mit einem Ankerrührer, Gaseinleitungsrohr, Thermometer und Rückflußkühler versehen ist, wurden 348 ml Benzin (Siedebereich 100-140°C), 175 ml Perchloräthylen und 3,28 g eines Polybutadienöls vom Molgewicht 1000 bis 1500 vorgelegt und bei einer Umdrehungsgeschwindigkeit von 60U/min eine Lösung von 85 g Dimethyl-diallylammoniumchlorid (60 %ige wäßrige Lösung), 29,5 g Glycidylmethacrylat, 4,5 g N-Vinylimidazol, 15 g Methylenbisacrylamid in 400 ml $H_2O$ und 0,57 g 2,2'-Azobis-(2-amidinopropan)dihydrochlorid [2,2'-Azobis(2-methylpropanimidamid)dihydrochlorid, $C_8H_{18}N_6 \cdot 2\ HCl$; Chemical Abstracts Registry Number Index: 2 997-92-4] in 60 Minuten zugetropft. Diese Monomerlösung wird in Form feiner Tröpfchen in der vorgelegten organischen Lösung verteilt.

Innerhalb von 30 Minuten wird das Bad auf 80°C und in weiteren 40 Minuten auf 100°C geheizt. Dann wird bei einer Badtemperatur von 90-91°C polymerisiert. Anschließend wird das $H_2O$ azeotrop abdestilliert und das entstandene Perlpolymerisat durch Absaugen isoliert und 2 x mit 100 ml Aceton gewaschen und bei 60°C und 200 Torr 8 Stunden getrocknet.

Die Ausbeute beträgt 95,3 g (95,3 % d.Th.).

Ein Produkt gleicher Oberflächenstruktur erhält man, wenn man 15 g Methylenbismethacrylamid statt 15 g Methylenbisacrylamid einsetzt.

Beispiel 2:

a) Man verfährt gemäß Beispiel 1, läßt jedoch eine Lösung aus 85 g Dimethyl-diallyl-ammoniumchlorid (60 %ige wäßrige Lösung), 34 g Glycidylmethacrylat, 15 g Methylenbisacrylamid in 400 ml Wasser und 0,57 g 2,2'-Azo-bisacrylamid in die vorgelegte organische Lösung tropfen.

Es wird innerhalb 30 Minuten auf 80°C aufgeheizt und bei 75-80°C polymerisiert.

Man erhält eine Ausbeute von 38,5 g Copolymerisat (98,5 % d.Th.).

b) Verfährt man gemäß Beispiel 2a, läßt jedoch eine Lösung von 85 g Dimethyl-diallylammoniumchlorid (60 %ige wäßrige Lösung), 25 g Glycidylmethacrylat, 9 g N-Vinylimidazol, 15 g Bisacrylamidoessigsäure in 168,4 ml Wasser und 0,85 2,2'-Azobis-(2-amidinopropan) dihydrochlorid in die vorgelegte organische Lösung tropfen, so erhält man ein Produkt, daß in der Qualität vergleichbar mit dem Produkt aus Beispiel 2a ist.

Beispiel 3:

Man verfährt gemäß Beispiel 1 tropft jedoch unter Einleitung von Stickstoff eine Lösung von 85 g Dimethyl-diallylammoniumchlorid (60 %ige wäßrige Lösung) 8,74 g Vinylcyclohexenmonoxyd, 9 g N-Vinylimidazol, 15 g Methylenbisacrylamid in 350 ml Wasser und 0,57 g 2,2'-Azobis-2-(amidinopropan)-dihydrochlorid innerhalb von 60 Minuten in die vorgelegte organische Lösung.
Man heizt auf eine Temperatur von 85°C und polymerisiert bei 78-81°C. Anschließend wird 30 Minuten bei 80-85°C nachgerührt.
Man erhält eine Ausbeute von 70,4 g Copolymerisat (70,9 % d.Th.).

Beispiel 4:

a) Man verfährt gemäß Beispiel 1, gibt jedoch 2,85 g

1,7-Bis-(2-hydroxypropyl-)1,1,3,3,5,5,7,7,-octamethyl-tetra-siloxan-bisacrylat statt des Polybutadienpolymeren in die vorgelegte organische Lösung. Unter Einleiten von Stickstoff tropft man eine Lösung von 85 g Dimethyl-di-allylammoniumchlorid (60 %ige wäßrige Lösung), 25 g Glycidylmethacrylat, 9 g N-Vinylimidazol, 15 g Methylenbisacrylamid in 400 ml Wasser und 0,57 g 2,2'-Azobis- (2-amidinopropan)dihydrochlorid innerhalb von 60 Minuten in die vorgelegte organische Lösung.

Über einen Zeitraum von 30 Minuten wird auf 83-85°C hochgeheizt und polymerisiert.

Man erhält eine Ausbeute von 100 g (100 % d.Th.).

b) Man erhält ein Copolymerisat von der gleichen Qualität, wenn 15 g Polyäthylenglykol-600-dimethacrylat statt Methylenbisacrylamid und 0,57 g Azobis-isobutyronitril statt 2,2'-Azobis-(2-amidinopropan)-dihydrochlorid eingesetzt werden.

Beispiel 5:

a) Man verfährt gemäß Beispiel 1, legt jedoch in der organischen Lösung statt der Polybutadienpolymeren ein Mischpolymerisat aus Polybutadienöl und Maleinsäureanhydrid vor, das unter Stickstoffatmosphäre bei 200°C hergestellt wird ( Lithene PM4, Fa. Rivertex Ltd., GB.):

Unter Einleiten von Stickstoff tropft man eine Lösung von 85 g Dimethyl-diallyl-ammoniumchlorid (60 %ige wäßrige Lösung), 25 g Vinylcyclohexenmonoxyd, 9 g Vinylimidazol und 15 g Bisacrylamidoessigsäure in 150 ml Wasser und 0,57 g 2,2'-Azobis-(2-amidinopropan)-dihydrochlorid innerhalb von 30 Minuten in die vorgelegte organische Lösung.

Über einen Zeitraum von 30 Minuten wird auf 92°C hochgeheizt und bei einer Temperatur von 84-85°C polymerisiert und 60 Minuten nachgeheizt.

Man erhält eine Ausbeute von 84,4 g (84,4 % d.Th.).

b) Man erhält ein Copolymerisat der gleichen Qualität, wenn man eine Lösung aus 68 g Dimethyl-diallyl-ammoniumchlorid (60 %ige wäßrige Lösung), 25 g Glycidylmethacrylat, 9 g N-Vinyl-N-methylacetamid und 19 g N,N-Methylenbisacrylamid in 30 ml Wasser in die vorgelegte organische Lösung tropft.

Beispiel 6:

a) Man verfährt gemäß Beispiel 1, tropft jedoch unter Einleiten von Stickstoff eine Lösung von 85 g Dimethyldiallylammoniumchlorid (60 %ige wäßrige Lösung), 25 g Allylglycidylether, 9 g N-Vinylimidazol und 15 g Methylenbisacrylamid in 350 ml $H_2O$ und 0,57 g Azoisobutyronitril innerhalb von 60 Minuten in die vorgelegte organische Lösung.
Über einen Zeitraum von 30 Minuten wird auf 80°C hochgeheizt und bei einer Temperatur von 77-79°C polymerisiert und 30 Minuten nachgeheizt.
Man erhält 68,3 g Copolymerisat (72 % d.Th.).

b) Man erhält ein Produkt gleicher Qualität, wenn man eine Lösung aus 69 g Dimethyl-diallylammoniumchlorid (60 %-ige wäßrige Lösung), 25 g Glycidylmethacrylat, 9 g N-Vinylformamid und 15 g Methylenbisacrylamid in 10 ml Wasser und 0,456 g 2,2'-Azobis-(2-amidinopropan)-dihydrochlorid in die vorgelegte organische Lösung tropft und innerhalb von 30 Minuten bei 75-88°C polymerisiert.

Beispiel 7:

Man verfährt gemäß Beispiel 1, tropft jedoch unter Einleiten von Stickstoff eine Lösung aus 75 g Glycidylmethacrylat, 16,7 g Dimethyl-diallylammoniumchlorid (60 %ige wäßrige Lösung) und 15 g Methylenbisacrylamid in 650 ml Wasser und 0,57 g 2,2'-Azobis-(2-amidinopropan)dihydrochlorid innerhalb von 30 Minuten bei einer Rührgeschwindigkeit von 100 U/min in die vorgelegte organische Lösung.

Über einen Zeitraum von 30 Minuten wird auf 90°C hochgeheizt und bei 78-81°C polymerisiert.
Man erhält 97 g Copolymerisat (97 % d.Th.)

Beispiel 8:

Man verfährt gemäß Beispiel 1, legt jedoch 3,28 g 1,7-Bis-(2-hydroxypropyl)-1,1,3,3,5,5,7,7-octamethyl-tetrasiloxanbisacrylat statt des Polybutadienöls in der organischen Lösung vor. Unter Einleiten von Stickstoff tropft man bei einer Rührgeschwindigkeit von 100 U/min. eine Lösung von 85 g Dimethyl-diallylammoniumchlorid(60 %ige wäßrige Lösung), 25 g Limonenoxyd, 9 g N-Vinylimidazol und 15 g Methylenbisacrylamid in 350 ml $H_2O$ und 0,57 g 2,2'-Azobis-(2-amidinopropan)dihydrochlorid innerhalb von 30 Minuten in die vorgelegte organische Lösung.

Über einen Zeitraum von 60 Minuten wird auf 90°C hochgeheizt, bei 75-76°C polymerisiert und 30 Minuten nachgeheizt.
Man erhält eine Ausbeute von 73 g Copolymerisat (73 % d.Th.).

Beispiel 9:

In einem 3-Liter-4-halskolben, der mit einem Ankerrührer, Gasleitungsrohr, Thermometer und Rückflußkühler versehen ist, wurden in 522 ml Benzin (Siedebereich 100°bis 140°C),

263 ml Perchloräthylen und 14,76 g eines Polybutadienöls vorgelegt. Bei einer Umdrehungsgeschwindigkeit von 120 U/min wird eine Monomerlösung von 105 g Glycidylmethacrylat, 7,5 g N-Vinylimidazol, 25 g Diallyl-dimethyl-ammoniumchlorid (60 %ige wäßrige Lösung), 22,5 g Methylenbisacrylamid in 1050 ml $H_2O$ und 0,855 g 2,2'-Azobis-(2-amidinopropan)-dihydrochlorid über den Tropftrichter zugegeben. Diese Monomer Lösung wird dabei in Form feiner Tröpfchen verteilt.

Innerhalb von 30 Minuten wird auf 90°C geheizt und bei 80-81°C polymerisiert. Anschließend wird 2 Stunden nachgerührt und das $H_2O$ in 5 Stunden azeotrop abdestilliert. Das erhaltene perlförmige Copolymerisat wird abgetrennt, mit Aceton gewaschen und bei 50°C und 266 mbar 6 Stunden getrocknet. Man erhält 149 g Copolymerisat (99,3 % d.Th.).

Beispiel 10:

Aktivierung des Trägers:

4 g Copolymerisat aus Beispiel 1, 4, 5 sowie 66 ml Dimethoxyethan und 3 g 1,4-Diaminobutan werden 3 h auf 80°C erhitzt. Nach dem Abkühlen wird der Träger abgefiltert, mit Wasser aminfrei gewaschen und anschließend in einer Lösung aus 100 ml 0,1 molarem Phosphatpuffer (pH 7,0) und 2,4 ml Glutardialdehyd (50 %ige wässrige Lösung) 1,5 Stunden bei Raumtemperatur aktiviert. Das Polymerisat wird abgetrennt und mit 1 l destilliertem Wasser gewaschen.

Enzym-Fixierung:

2 g aktivierter Träger werden in 30 ml 0,1 molarem Phosphatpuffer (pH 7,0) mit 200 mg Lipase aus Candida cylindracea (Fa. Sigma) 16 Stunden bei Raumtemperatur gerührt, anschließend abfiltriert und mit destilliertem Wasser, 0,5

molarer Natriumchloridlösung und 0,1 molarem Phosphatpuffer (pH 7,0) gewaschen. Die gefundenen Enzymaktivitäten sind in der nachfolgenden Tabelle aufgelistet.

Beispiel 11:

Man verfährt gemäß Beispiel 10, setzt jedoch statt 1,4-Diaminobutan die gleiche Menge 1,6-Diaminohexan ein und immobilisiert Lipase aus Candida cylindracea an das Copolymerisat aus Beispiel 2 oder 5. Die Ergebnisse sind in nachfolgender Tabelle wiedergegeben.

Beispiel 12:

2 g Copolymerisat aus Beispiel 1 oder 4 in 50 ml 0,1 molarem Phosphatpuffer (pH 7,5) und 200 mg Lipase aus Candida cylindracea werden 16 Stunden bei Raumtemperatur gerührt. Der Träger wird abgetrennt und nacheinander mit Wasser, 0,5 molarer Natriumchloridlösung und wieder Wasser gewaschen.

Fixierung von Lipase aus Candida cylindracea:

| Copolymerisat | Fixierungsart | Units/g Träger |
|---|---|---|
| Beispiel 2 | Beispiel 10 | 151 |
| 5 | 10 | 119 |
| 6 | 10 | 151 |
| 5 | 11 | 120 |
| 2 | 11 | 150 |
| 1 | 12 | 92 |
| 4 | 12 | 75 |
| 9 | 11 | 220 |

1 Unit Enzym hydrolisiert in einer Minute 1 µMol Carbonsäureester.

Beispiel 13:

Analog Beispiel 10 werden 100 mg Lipase aus Rhizopus Sp. an 2 g der Copolymerisate aus Beispiel 2 und 5 immobilisiert. Beide Fixierungen zeigen Lipaseaktivitäten von 35 U/g Träger

Beispiel 14:

200 µl Schweineleberesterase die in 3,2 molarer Ammoniumsulfatlösung suspendiert ist, werden vor der Fixierung 48 Stunden bei 7°C gegen 1 molaren Phosphatpuffer (pH 7,5) dialysiert. Anschließend wird das gereinigte Enzym analog Beispiel 10 an ein Copolymerisat aus Beispiel 7 fixiert. Die Fixierung ergibt eine Esteraseaktivität von 15 U/g Träger.

Beispiel 15:

Wie in Beispiel 10 beschrieben wird Lipase aus Schweinepankreas an das Copolymerisat aus Beispiel 9 fixiert. Die Fixierung ergibt eine Lipaseaktivität von 95 U/g Träger.

Patentansprüche:

1) Ein vernetztes, poröses, perlförmiges Copolymerisat mit

a) wiederkehrenden Einheiten der monomeren Verbindung der allgemeinen Formel I,

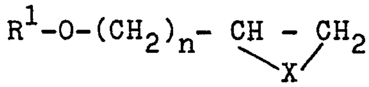 I

in der $R^1$ $(C_2-C_4)$ Alkenyl oder die korrespondierende Säure,
X Sauerstoff oder NH und
n 1-6 bedeuten,

oder wiederkehrenden Einheiten der monomeren Verbindung der allgemeinen Formel II,

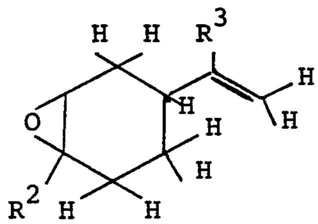 II

in der $R^2$ und $R^3$ unabhängig voneinander Wasserstoff oder Methyl bedeuten,

oder mehrerer solcher Verbindungen und

b) wiederkehrenden Einheiten eines oder mehrerer monomerer n-valenter Vernetzter und

c) wiederkehrenden Einheiten der monomeren Verbindung der allgemeinen Formel III,

$CH_2$=CH-N($R^4$)-C($R^5$)=O III

in der $R^4$ und $R^5$ unabhängig voneinander Wasserstoff oder $(C_1-C_3)$-Alkyl bedeuten oder zusammen für $-(CH_2)_n-$ mit n = 3, 4 oder 5 stehen,

oder mehrerer derartiger Verbindungen und

d) wiederkehrenden Einheiten der monomeren Verbindung der allgemeinen Formel IV,

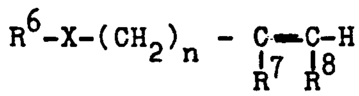

IV

in der $R^6$ den Rest einer heterozyklischen 5-Ringverbindung, die im Ring mindestens ein protonierbares Stickstoffatom besitzt, oder Pyridyl, Chinolyl, Isochinolyl oder Pyrazinyl und

$R^7$ und $R^8$ Wasserstoff oder $(C_1-C_4)$-Alkyl und

X Sauerstoff oder Schwefel oder eine direkte Bindung und

n eine Zahl von 0 bis 4 bedeuten,

oder mehrerer derartiger Verbindungen und

e) wiederkehrenden Einheiten der monomeren Verbindung der allgemeinen Formel V,

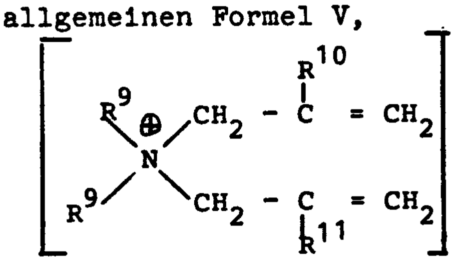

V

in der $R^9$ eine $(C_1-C_{10})$-Alkylgruppe,

$R^{10}$ und $R^{11}$ unabhängig voneinander Wasserstoff oder Methyl und

Y Halogen bedeuten,

oder mehrere derartige Verbindungen.

2) Copolymerisat nach Anspruch 1, dadurch gekennzeichnet, daß das Copolymerisat aus 10-90 Gew.-% wiederkehrender

Einheiten der Verbindung der allgemeinen Formel I oder II, 0,05-50 Gew.-% wiederkehrender Einheiten des n-valenten Vernetzers, 0-50 Gew.-% wiederkehrender Einheiten der Verbindung der allgemeinen Formel III, 0-90 Gew.-% wiederkehrender Einheiten der Verbindung der allgemeinen Formel IV und 9,5-80 Gew.-% wiederkehrender Einheiten der Verbindung der allgemeinen Formel V besteht.

3) Copolymerisat nach Anspruch 2, dadurch gekennzeichnet, daß das Copolymerisat aus 25-75 Gew.-% wiederkehrender Einheiten der Verbindung der allgemeinen Formel I oder II, 0,1-35 Gew.-% wiederkehrender Einheiten des n-valenten Vernetzers, 5-25 Gew.-% wiederkehrender Einheiten der Verbindung der allgemeinen Formel III, 10-80 Gew.-% wiederkehrender Einheiten der Verbindung der allgemeinen Formel IV und 10-50 Gew.-% wiederkehrender Einheiten der Verbindung der allgemeinen Formel V besteht.

4) Copolymerisat nach einem oder mehreren der Ansprüche 1 bis 3, dadurch gekennzeichnet, daß es als Vernetzer Einheiten von N,N'-Methylen-bis-acrylamid und/oder N,N'-Methylen-bis-methacrylamid und/oder Ethylenglykol-diacrylat und/oder Ethylenglykol-dimethacrylat und/oder Di-ethylenglykol-diacrylat und/oder Diethylenglykoldimeth-acrylat und/oder Polyethylenglykol 200 bis 600-diacrylat und/oder Polyethylenglykol 200 bis 600-dimethacrylat und/oder N,N',N"-Tris-(2-acryloyloxy-ethyl)-isocyanurat und/der N,N',N"-Tris-(2-methacryloyl-oxy-ethyl)-isocyanurat enthält.

5) Verfahren zur Herstellung des Copolymerisats nach Anspruch 1, dadurch gekennzeichnet, daß eine oder mehrere Verbindungen der allgemeinen Formel I oder II, eine oder mehrere n-valente Vernetzer, eine oder mehrere Verbindungen der allgemeinen Formel III, eine oder mehrere Verbindungen der allgemeinen Formel IV und eine oder mehrere Verbindungen der allgemeinen Formel V nach dem Verfahren der umgekehrten Suspensionspolymerisation copolymerisiert werden.

6) Verfahren nach Anspruch 5, dadurch gekennzeichnet, daß 10-90 Gew.-% einer oder mehrerer Verbindungen der allgemeinen Formel I oder II, 0,05-50 Gew.-% eines oder mehrerer n-valenter Vernetzer, 0-50 Gew.-% einer oder mehrerer Verbindungen der allgemeinen Formel III, 0-90 Gew.-% einer oder mehrerer Verbindungen der allgemeinen Formel IV und 9,5-80 Gew.-% einer oder mehrerer Verbindungen der allgemeinen Formel V eingesetzt werden.

7) Verfahren nach Anspruch 6, dadurch gekennzeichnet, daß 25-75 Gew.-% einer oder mehrerer Verbindungen der allgemeinen Formel I oder II, 0,1-35 Gew.-% eines oder mehrerer n-valenter Vernetzer, 5-25 Gew.-% einer oder mehrerer Verbindungen der allgemeinen Formel III, 10-80 Gew.-% einer oder mehrerer Verbindungen der allgemeinen Formel IV und 10-50 Gew.-% einer oder mehrerer Verbindungen der allgemeinen Formel V eingesetzt werden.

8) Verwendung des Copolymerisats nach Anspruch 1-5 als Enzymträger.

9) Verfahren zur Immobilisierung von Enzymen aus wäßriger Lösung durch kovalente Bindung an einen polymeren Träger, dadurch gekennzeichnet, daß als polymerer Träger ein Copolymerisat nach Anspruch 1-5 eingesetzt wird.

10) Verfahren nach Anspruch 9, dadurch gekennzeichnet, daß das Copolymerisat vor der Fixierung aktiviert wird.

Patentansprüche Österreich und Spanien:

1) Verfahren zur Herstellung eines vernetzten, porösen, perlförmigen Copolymerisats, dadurch gekennzeichnet, daß eine oder mehrere Verbindungen der allgemeinen Formel I oder II,

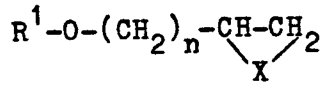 I

in der $R^1$ $(C_2-C_4)$ Alkenyl oder die korrespondierende Säure,
X Sauerstoff oder NH und
n 1-6 bedeuten,

oder wiederkehrenden Einheiten der monomeren Verbindung der allgemeinen Formel II,

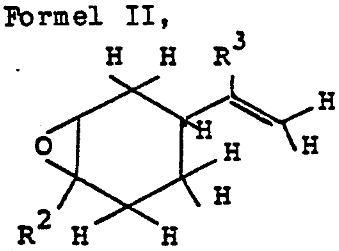 II

in der $R^2$ und $R^3$ unabhängig voneinander Wasserstoff oder Methyl bedeuten,

eine oder mehrere n-valente Vernetzer,

eine oder mehrere Verbindungen der allgemeinen Formel III,

$$CH_2=CH-N(R^4)-C(R^5)=O \quad \text{III}$$

in der $R^4$ und $R^5$ unabhängig voneinander Wasserstoff oder $(C_1-C_3)$-Alkyl bedeuten oder zusammen für $-(CH_2)_n-$ mit n = 3, 4 oder 5 stehen,

eine oder mehrere Verbindungen der allgemeinen Formel IV

$$R^6-X-(CH_2)_n-\underset{R^7}{\underset{|}{C}} = \underset{R^8}{\underset{|}{C}}-H \qquad IV$$

in der $R^6$ den Rest einer heterozyklisschen 5-Ringverbindung, die im Ring mindestens ein protonierbares Stickstoffatom besitzt, oder Pyridyl, Chinolyl, Isochinolyl oder Pyrozinyl und

$R^7$ und $R^8$ Wasserstoff oder $(C_1-C_4)$-Alkyl und

X Sauerstoff oder Schwefel oder eine direkte Bindung und

n eine Zahl von 0 bis 4 bedeuten,

und eine oder mehrere Verbindungen der allgemeinen Formel V

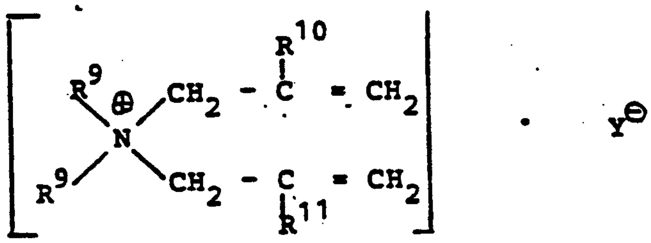

V

in der ein $R^9$ eine $(C_1-C_{10})$-Alkylgruppe,

$R^{10}$ und $R^{11}$ unabhängig voneinander Wasserstoff oder Methyl und

Y Halogen bedeuten,

nach dem Verfahren der umgekehrten Suspensionspolymerisation copolymerisiert werden.

2) Verfahren nach Anspruch 1, dadurch gekennzeichnet, daß 10-90 Gew.-% einer oder mehrerer Verbindungen der allgemeinen Formel I oder II, 0,05-50 Gew.-% eines oder mehrerer n-valenter Vernetzer, 0-50 Gew.-% einer oder mehrerer Verbindungen der allgemeinen Formel III, 0-90 Gew.-% einer oder mehrerer Verbindungen der allgemeinen Formel IV und 9,5-80 Gew.-% einer oder mehrerer Verbindungen der allgemeinen Formel V eingesetzt werden.

3) Verfahren nach Anspruch 1, dadurch gekennzeichnet, daß 25-75 Gew.-% einer oder mehrerer Verbindungen der allgemeinen Formel I oder II, 0,1-35 Gew.-% eines oder mehrerer n-valenter Vernetzer, 5-25 Gew.-% einer oder mehrerer Verbindungen der allgemeinen Formel III, 10-80 Gew.-% einer oder mehrerer Verbindungen der allgemeinen Formel IV und 10-50 Gew.-% einer oder mehrerer Verbindungen der allgemeinen Formel V eingesetzt werden.

4) Verfahren zur Immobilisierung von Enzymen aus wäßriger Lösung durch kovalente Bindung an einen polymeren Träger, dadurch gekennzeichnet, daß als polymerer Träger ein Copolymerisat eingesetzt wird, das nach einem oder mehreren der Ansprüche 1 bis 3 erhältlich ist.

5) Verfahren nach Anspruch 4, dadurch gekennzeichnet, daß das Copolymerisat vor der Fixierung aktiviert wird.

Europäisches Patentamt

# EUROPÄISCHER RECHERCHENBERICHT

0220593

Nummer der Anmeldung

EP 86 11 4183

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 070 348 (D.KRÄEMER et al.)<br>* Patentanspruch 1; Spalte 9, Zeilen 56-65 * | 1-5 | C 08 F 246/00<br>C 08 F 226/04<br>C 12 N 11/08 |
| A | EP-A-0 058 767 (RÖHM GmbH)<br>* Patentansprüche 1,8 * | 1,5 | |
| A | EP-A-0 088 964 (B.A.S.F.)<br>* Patentansprüche 1,14 * | 1-5 | |
| A | DE-A-2 252 866 (AGENCY OF INDUSTRIAL SCIENCE AND TECHNOLOGY)<br>* Patentanspruch 1 * | 1-3 | |
| | | | **RECHERCHIERTE SACHGEBIETE (Int. Cl.4)** |
| | | | C 08 F<br>C 12 N |

Der vorliegende Recherchenbericht wurde für alle Patentansprüche erstellt.

| Recherchenort | Abschlußdatum der Recherche | Prüfer |
|---|---|---|
| DEN HAAG | 29-01-1987 | CAUWENBERG C.L.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82